# EUROPEAN PATENT APPLICATION

(11) **EP 4 628 082 A1**
(43) Date of publication of application: **08.10.2025**
(21) Application number: 23902838.4
(22) Date of filing: 15.12.2023
(51) Int. Cl.: A61K 31/485, A61K 38/07, A61P 25/04, A61P 17/04

(54) **PHARMACEUTICAL COMPOSITION OF KAPPA OPIOID RECEPTOR AGONIST, PREPARATION METHOD THEREFOR, AND USE THEREOF**

(30) Priority: 15.12.2022 CN 202211613923
(71) Applicant: Yichang Humanwell Pharmaceutical Co., Ltd., Yichang, Hubei 443005 (CN)
(72) Inventor: HUANG, Minglai, Yichang, Hubei 443005 (CN); YUAN, Jing, Yichang, Hubei 443005 (CN); ZHOU, Hao, Yichang, Hubei 443005 (CN); YANG, Jun, Yichang, Hubei 443005 (CN); LI, Lie, Yichang, Hubei 443005 (CN); LIAO, Subo, Yichang, Hubei 443005 (CN); LONG, Qiaoqiao, Yichang, Hubei 443005 (CN); BIAN, Di, Yichang, Hubei 443005 (CN); TANG, Lihua, Yichang, Hubei 443005 (CN); XIE, Tianpeng, Yichang, Hubei 443005 (CN); LV, Jinliang, Yichang, Hubei 443005 (CN); LIU, Rong, Yichang, Hubei 443005 (CN); HE, Yao, Yichang, Hubei 443005 (CN); LIAO, Zongquan, Yichang, Hubei 443005 (CN)
(74) Representative: WSL Patentanwälte Partnerschaft mbB
(86) International application number: PCT/CN2023/139232
(87) International publication number: WO 2024/125645

(57) **Abstract**

Provided are a pharmaceutical composition of a kappa opioid receptor agonist, a preparation method therefor, and use thereof. The pharmaceutical composition comprises a compound represented by formula (I), a stereoisomer thereof, a metabolite thereof, or a pharmaceutically acceptable salt thereof, and an optional osmotic pressure regulator, adjusted to pH 3.0-5.0 with hydrochloric acid. The pharmaceutical composition is good in stability, has a preparation process suitable for industrial production with a simple formula, and is compatible with a clinical administration route.

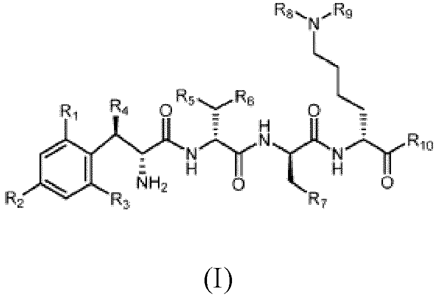

## Description

This application claims priority to a Chinese patent application filed with the Chinese Patent Office on December 15, 2022, application number 202211613923.5 and the invention title "PHARMACEUTICAL COMPOSITION OF KAPPA OPIOID RECEPTOR AGONIST, PREPARATION METHOD THEREFOR, AND USE THEREOF", the contents of which should be understood to be incorporated herein by reference.

### Technical Field

The present application relates to, but is not limited to, the technical field of pharmaceutical formulation, in particular to a pharmaceutical composition of a kappa opioid receptor agonist, a preparation method and use thereof.

### Background

A kappa opioid receptor (KOR) is a member of the opioid receptor family, which is mainly distributed in the claustrum, cerebral cortex, hypothalamus, piriform nucleus, nucleus accumbens, caudate putamen, substantia nigra and dorsal horn of spinal cord. Activation of kappa opioid receptors can break down G protein into Gα and Gβγ subunits, thus affecting adenylyl cyclase, K⁺/Ca²⁺ channel activity, phospholipase C, and p42/44 mitogen-activated protein (MAP) kinase pathway. Many physiological processes are related to the activation of kappa opioid receptors, including analgesia, antipruritic, diuresis, etc.

WO2021262173A1 discloses a new class of peptide compounds with peripherally selective kappa opioid receptor agonistic activity, as represented by the following formula. Preclinical studies have shown that the peptide compounds can significantly improve adverse reactions such as respiratory depression and addiction of opioids while having pharmacological activities such as analgesia and antipruritic.

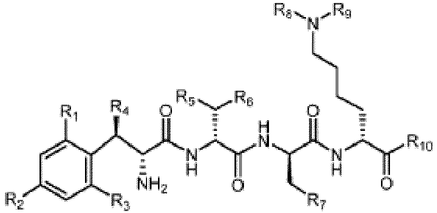

Therefore, there is an urgent clinical need to develop pharmaceutical formulations with good stability of such compounds and suitable for clinical administration routes.

### Summary

In a first aspect, the present application provides a pharmaceutical composition of a kappa opioid receptor agonist, which comprises a compound represented by formula (I), or a stereoisomer thereof, or a metabolite thereof or a pharmaceutically acceptable salt thereof, optionally an osmotic pressure regulator, and a pH adjusting agent and water for injection;
wherein the compound represented by formula (I) is: 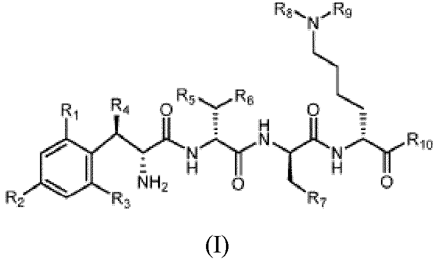 wherein:
R₁, R₂, and R₃ are independently selected from the group consisting of H, CN, Cl, F, unsubstituted C₁-C₈ alkyl, substituted C₁-C₈ alkyl, unsubstituted C₃-C₁₀ cycloalkyl, and substituted C₃-C₁₀ cycloalkyl;
R₄ is selected from H, unsubstituted C₁-C₈ alkyl, substituted C₁-C₈ alkyl, unsubstituted C₃-C₁₀ cycloalkyl, and substituted C₃-C₁₀ cycloalkyl;
R₅ and R₆ are independently selected from H, unsubstituted C₁-C₈ alkyl, substituted C₁-C₈ alkyl, unsubstituted C₃-C₁₀ cycloalkyl, substituted C₃-C₁₀ cycloalkyl, unsubstituted aryl, substituted aryl, unsubstituted heterocyclyl, and substituted heterocyclyl;
R₇ is selected from H, unsubstituted C₁-C₈ alkyl, substituted C₁-C₈ alkyl, unsubstituted C₃-C₁₀ cycloalkyl, and substituted C₃-C₁₀ cycloalkyl;
R₈ and R₉ are independently selected from H, unsubstituted C₁-C₈ alkyl, substituted C₁-C₈ alkyl, unsubstituted C₃-C₁₀ cycloalkyl, O-substituted C₁-C₈ alkyl, O-unsubstituted C₁-C₈ alkyl, CH₃O(CH₂CH₂)ₙCH₂CH₂-, and HO(CH₂CH₂)ₙCH₂CH₂-;
R₁₀ is 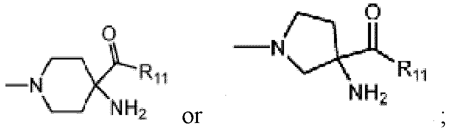
R₁₁ is OR₁₂ or NR₁₃R₁₄;
R₁₂ is selected from H, unsubstituted C₁-C₂₄ alkyl, substituted C₁-C₂₄ alkyl, O-substituted C₁-C₂₄ alkyl, O-unsubstituted C₁-C₂₄ alkyl, CH₃O(CH₂CH₂)ₙCH₂CH₂-, and HO(CH₂CH₂)ₙCH₂CH₂-;
R₁₃ and R₁₄ are independently selected from H, unsubstituted C₁-C₂₄ alkyl, substituted C₁-C₂₄ alkyl, O-substituted C₁-C₂₄ alkyl, O-unsubstituted C₁-C₂₄ alkyl, CH₃O(CH₂CH₂)ₙCH₂CH₂-, and HO(CH₂CH₂)ₙCH₂CH₂-; and
n is an integer from 0 to 100;
the pH adjusting agent is hydrochloric acid; and pH of the pharmaceutical composition is 3.0-5.0.

In another aspect, the present application provides a method for preparing the aforementioned pharmaceutical composition of a kappa opioid receptor agonist.

In a third aspect, the present application provides a kappa opioid receptor agonist which is an acetate salt of a compound as represented by formula (II):

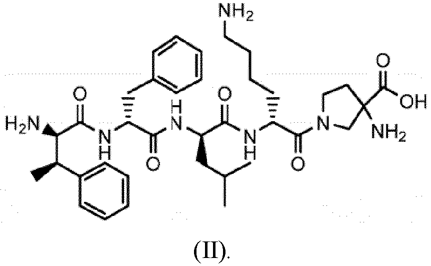

In a fourth aspect, the present application provides the aforementioned pharmaceutical composition of a kappa opioid receptor agonist or the acetate salt of the compound represented by formula (II) for use in preventing, treating, or treating and preventing a disease associated with a kappa opioid-like substance receptor.

In a fifth aspect, the present application provides use of the aforementioned pharmaceutical composition of a kappa opioid receptor agonist or the acetate salt of the compound represented by formula (II) in the preparation of a medicament for preventing, treating, or treating and preventing a disease associated with a kappa opioid-like substance receptor.

In a sixth aspect, the present application provides a method for preventing, treating, or treating and preventing a disease associated with a kappa opioid-like substance receptor, which comprises administering to an individual in need thereof a therapeutically effective amount of the aforementioned pharmaceutical composition of a kappa opioid receptor agonist or the acetate salt of the compound represented by formula (II).

Additional features and advantages of the present application will be set forth in the description which follows, and in part will become apparent from the description, or may be learned by practice of the application. Other advantages of the present application may be realized and obtained by the solutions described in the specification.

### Brief Description of Drawings

The accompanying drawings are used to provide an understanding of the technical solutions of the present application, and constitute a part of the specification. They are used to explain the technical solutions of the present application together with the embodiments of the present application, and do not constitute a limitation to the technical solutions of the present application.

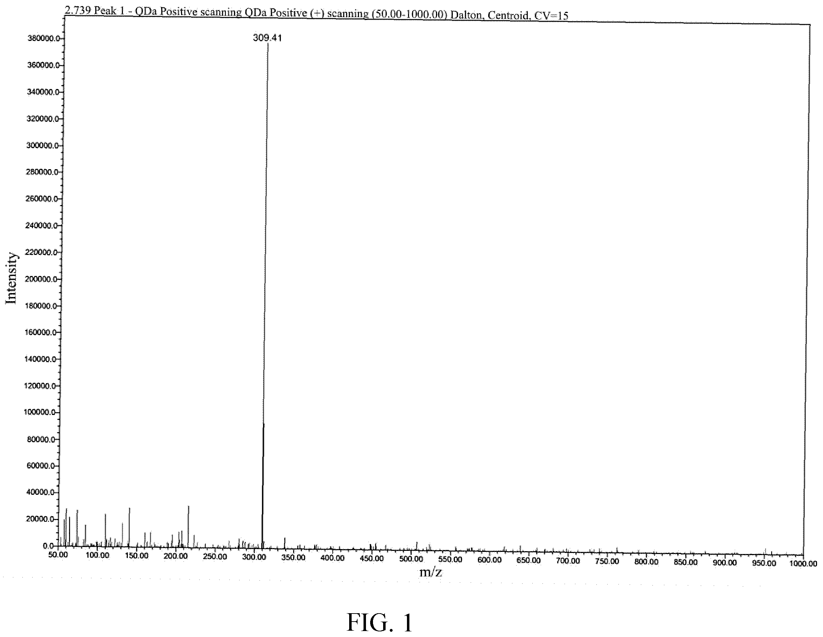

FIG. 1 is a mass spectrum of flocculent precipitated impurities.

### Detailed Description

In their studies, the present inventors have found that peptide compounds with peripherally selective kappa opioid receptor agonistic activity are easily decomposed by peptide hydrolase and pepsin after oral administration into animals, resulting in low oral bioavailability. In addition, during the study of liquid formulations, such type of compounds are found that when a representative compound (II) thereof is prepared into a conventional injection, after sterilization according to the conventional requirements of injections, a flocculent precipitate is separated out when the sample is cooled to room temperature. After the cooled pharmaceutical solution is centrifuged at 3000 rpm to 10000 rpm, the precipitate is collected and detected by mass spectrometry (mass spectrometry conditions: chromatographic column: ACQUITY UPLC BEH C8, 50 mm × 2.1 mm, 1.7 µm (20180306-C8-08); mobile phase A: 0.1% formic acid/water; mobile phase B: 0.1% formic acid/acetonitrile; flow rate: 0.2 ml/min; column temperature: 30 °C; detector wavelength: UV-210 nm; QDa; cone voltage: positive scan 10 volts, negative scan 5 volts; capillary voltage: plus 1.5 kV, minus 0.5 kV). Based on the detected MS values, it is speculated that the white flocculent precipitate is a degradation impurity as shown in the following formula:

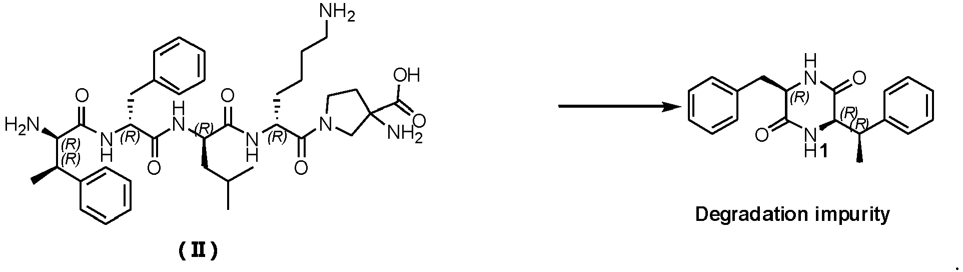

In view of this, the present application provides a pharmaceutical composition of a kappa opioid receptor agonist and a preparation method therefor. The pharmaceutical composition of a kappa opioid receptor agonist has simple composition, good stability, and no precipitation in high-temperature sterilization process, and is compatible with clinical administration route.

In a first aspect, the present application provides a pharmaceutical composition of a kappa opioid receptor agonist, which comprises a compound represented by formula (I), or a stereoisomer thereof, or a metabolite thereof or a pharmaceutically acceptable salt thereof, optionally an osmotic pressure regulator, and a pH adjusting agent and water for injection;
wherein the compound represented by formula (I) is: 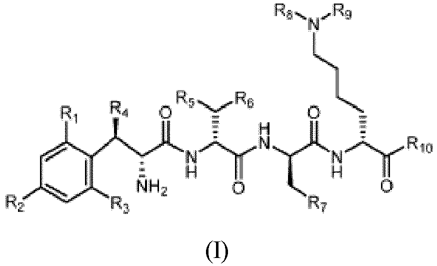 wherein:
R₁, R₂, and R₃ are independently selected from the group consisting of H, CN, Cl, F, unsubstituted C₁-C₈ alkyl, substituted C₁-C₈ alkyl, unsubstituted C₃-C₁₀ cycloalkyl, and substituted C₃-C₁₀ cycloalkyl;
R₄ is selected from **H,** unsubstituted C₁-C₈ alkyl, substituted C₁-C₈ alkyl, unsubstituted C₃-C₁₀ cycloalkyl, and substituted C₃-C₁₀ cycloalkyl;
R₅ and R₆ are independently selected from **H,** unsubstituted C₁-C₈ alkyl, substituted C₁-C₈ alkyl, unsubstituted C₃-C₁₀ cycloalkyl, substituted C₃-C₁₀ cycloalkyl, unsubstituted aryl, substituted aryl, unsubstituted heterocyclyl, and substituted heterocyclyl;
R₇ is selected from **H,** unsubstituted C₁-C₈ alkyl, substituted C₁-C₈ alkyl, unsubstituted C₃-C₁₀ cycloalkyl, and substituted C₃-C₁₀ cycloalkyl;
R₈ and R₉ are independently selected from **H,** unsubstituted C₁-C₈ alkyl, substituted C₁-C₈ alkyl, unsubstituted C₃-C₁₀ cycloalkyl, O-substituted C₁-C₈ alkyl, O-unsubstituted C₁-C₈ alkyl, CH₃O(CH₂CH₂)ₙCH₂CH₂-, and HO(CH₂CH₂)ₙCH₂CH₂-;
R₁₀ is 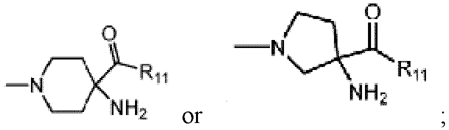
R₁₁ is OR₁₂ or NR₁₃R₁₄;
R₁₂ is selected from H, unsubstituted C₁-C₂₄ alkyl (which may be linear alkyl, linear alkyl, or cycloalkyl), substituted C₁-C₂₄ alkyl (which may be linear alkyl, linear alkyl, or cycloalkyl), O-substituted C₁-C₂₄ alkyl (which may be linear alkyl, linear alkyl, or cycloalkyl), O-unsubstituted C₁-C₂₄ alkyl (which may be linear alkyl, linear alkyl, or cycloalkyl), CH₃O(CH₂CH₂)ₙCH₂CH₂-, and HO(CH₂CH₂)ₙCH₂CH₂-;
R₁₃ and R₁₄ are independently selected from H, unsubstituted C₁-C₂₄ alkyl (which may be linear alkyl, linear alkyl, or cycloalkyl), substituted C₁-C₂₄ alkyl (which may be linear alkyl, linear alkyl, or cycloalkyl), O-substituted C₁-C₂₄ alkyl (which may be linear alkyl, linear alkyl, or cycloalkyl), O-unsubstituted C₁-C₂₄ alkyl (which may be linear alkyl, linear alkyl, or cycloalkyl), CH₃O(CH₂CH₂)ₙCH₂CH₂-, and HO(CH₂CH₂)ₙCH₂CH₂-; and
n is an integer from 0 to 100;
the pH adjusting agent is hydrochloric acid; and pH of the pharmaceutical composition is 3.0-5.0.

In some embodiments, the present application provides a pharmaceutical composition of a kappa opioid receptor agonist, which comprises a compound represented by formula (I), or a stereoisomer thereof, or a metabolite thereof or a pharmaceutically acceptable salt thereof, a pH adjusting agent, and water for injection. The pH adjusting agent is hydrochloric acid; and pH of the pharmaceutical composition is 3.0-5.0.

The present application provides a pharmaceutical composition of a kappa opioid receptor agonist, which comprises a compound represented by formula (I), or a stereoisomer thereof, or a metabolite thereof or a pharmaceutically acceptable salt thereof, an osmotic pressure regulator, a pH adjusting agent, and water for injection. The pH adjusting agent is hydrochloric acid; and pH of the pharmaceutical composition is 3.0-5.0.

In some embodiments, the compound of formula (I) is: 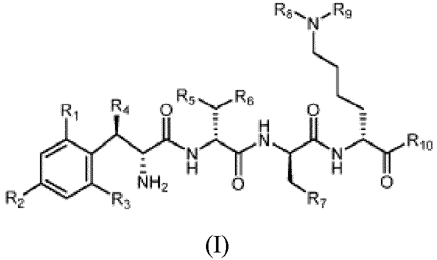 wherein:
R₁, R₂, and R₃ are independently selected from the group consisting of H, CN, Cl, F, unsubstituted C₁-C₈ alkyl, substituted C₁-C₈ alkyl, unsubstituted C₃-C₁₀ cycloalkyl, and substituted C₃-C₁₀ cycloalkyl;
R₄ is selected from H, unsubstituted C₁-C₈ alkyl, substituted C₁-C₈ alkyl, unsubstituted C₃-C₁₀ cycloalkyl, and substituted C₃-C₁₀ cycloalkyl;
R₅ and R₆ are independently selected from H, unsubstituted C₁-C₈ alkyl, substituted C₁-C₈ alkyl, unsubstituted C₃-C₁₀ cycloalkyl, substituted C₃-C₁₀ cycloalkyl, unsubstituted aryl, substituted aryl, unsubstituted heterocyclyl, and substituted heterocyclyl;
R₇ is selected from H, unsubstituted C₁-C₈ alkyl, substituted C₁-C₈ alkyl, unsubstituted C₃-C₁₀ cycloalkyl, and substituted C₃-C₁₀ cycloalkyl;
R₈ and R₉ are independently selected from H, unsubstituted C₁-C₈ alkyl, substituted C₁-C₈ alkyl, unsubstituted C₃-C₁₀ cycloalkyl, O-substituted C₁-C₈ alkyl, and O-unsubstituted C₁-C₈ alkyl;
R₁₀ is 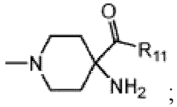
R₁₁ is OR₁₂ or NR₁₃R₁₄;
R₁₂ is selected from H, unsubstituted C₁-C₂₄ alkyl, substituted C₁-C₂₄ alkyl, O-substituted C₁-C₂₄ alkyl, O-unsubstituted C₁-C₂₄ alkyl, CH₃O(CH₂CH₂)ₙCH₂CH₂-, and HO(CH₂CH₂)ₙCH₂CH₂-;
R₁₃ and R₁₄ are independently selected from H, unsubstituted C₁-C₂₄ alkyl, substituted C₁-C₂₄ alkyl, O-substituted C₁-C₂₄ alkyl, O-unsubstituted C₁-C₂₄ alkyl, CH₃O(CH₂CH₂)ₙCH₂CH₂-, and HO(CH₂CH₂)ₙCH₂CH₂-; and
n is an integer from 0 to 100.

In some embodiments, in the compound of formula (I), wherein, R₁, R₂, and R₃ are independently selected from H, CN, Cl, F, unsubstituted C₁-C₄ alkyl, substituted C₁-C₄ alkyl, unsubstituted C₃-C₈ cycloalkyl, and substituted C₃-C₈ cycloalkyl;
R₄ is selected from unsubstituted C₁-C₄ alkyl, substituted C₁-C₄ alkyl, unsubstituted C₃-C₈ cycloalkyl, and substituted C₃-C₈ cycloalkyl;
R₅ and R₆ are independently selected from H, unsubstituted C₁-C₄ alkyl, substituted C₁-C₄ alkyl, unsubstituted C₃-C₈ cycloalkyl, substituted C₃-C₈ cycloalkyl, unsubstituted aryl, substituted aryl, unsubstituted heterocyclyl, and substituted heterocyclyl;
R₇ is selected from unsubstituted C₁-C₄ alkyl, substituted C₁-C₄ alkyl, unsubstituted C₃-C₈ cycloalkyl, and substituted C₃-C₈ cycloalkyl;
R₈ and R₉ are independently selected from H, unsubstituted C₁-C₄ alkyl, substituted C₁-C₄ alkyl, O-substituted C₁-C₄ alkyl, and O-unsubstituted C₁-C₄ alkyl;
R₁₀ is 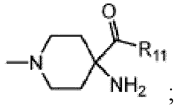
R₁₁ is selected from OR₁₂ and NR₁₃R₁₄;
R₁₂ is selected from H, unsubstituted C₁-C₁₂ alkyl, substituted C₁-C₁₂ alkyl, O-substituted C₁-C₁₂ alkyl, O-unsubstituted C₁-C₁₂ alkyl, CH₃O(CH₂CH₂)ₙCH₂CH₂-, and HO(CH₂CH₂)ₙCH₂CH₂-;
R₁₃ and R₁₄ are independently selected from H, unsubstituted C₁-C₁₂ alkyl, substituted C₁-C₁₂ alkyl, O-substituted C₁-C₁₂ alkyl, O-unsubstituted C₁-C₁₂ alkyl, CH₃O(CH₂CH₂)ₙCH₂CH₂-, and HO(CH₂CH₂)ₙCH₂CH₂-; and
n is an integer from 0 to 50.

In some embodiments, in the compound of formula (I), wherein, R₁, R₂, and R₃ are independently selected from H, Cl, F, methyl, ethyl, propyl, and isopropyl;
R₄ is selected from methyl, ethyl, propyl, isopropyl, cyclopropyl, cyclobutyl, cyclopentyl, and cyclohexyl;
R₅ and R₆ are independently selected from H, methyl, ethyl, n-propyl, isopropyl, cyclopropyl, cyclobutyl, cyclopentyl, cyclohexyl, and phenyl;
R₇ is selected from methyl, ethyl, propyl, isopropyl, cyclopropyl, cyclobutyl, cyclopentyl, and cyclohexyl;
R₈ and R₉ are independently selected from H, methyl, ethyl, propyl, and isopropyl;
R₁₀ is 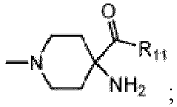
R₁₁ is selected from OR₁₂ and NR₁₃R₁₄;
R₁₂ is selected from H, methyl, ethyl, n-propyl, and isopropyl;
R₁₃ and R₁₄ are independently selected from H, methyl, ethyl, n-propyl, isopropyl, cyclopropyl, cyclobutyl, cyclopentyl, and cyclohexyl.

In some embodiments, in the compound of formula (I), wherein, R₁, R₂, R₃, R₅, R₈, and R₉ are H; R₄ is methyl; R₆ is phenyl; R₇ is isopropyl;
R₁₀ is 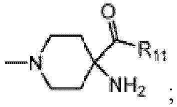
R₁₁ is OR₁₂; R₁₂ is H; and R₁₃, R₁₄, and n are absent, and the compound of formula (I) is a compound as represented by formula (IV):

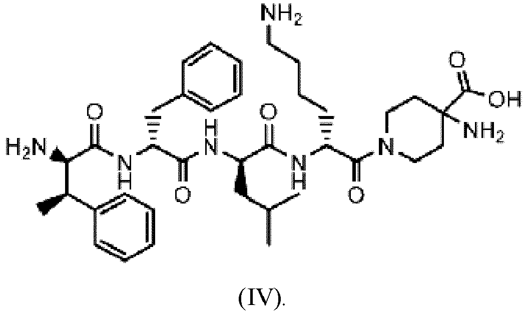

In some embodiments, in the compound of formula (I), wherein, R₁, R₂, R₃, R₅, R₈, and R₉ are H; R₄ is methyl; R₆ is phenyl; R₇ is isopropyl;
R₁₀ is 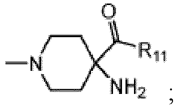
R₁₁ is OR₁₂; R₁₂ is Me; and R₁₃, R₁₄, and n are absent, and the compound of formula (I) is a compound as represented by formula (V):

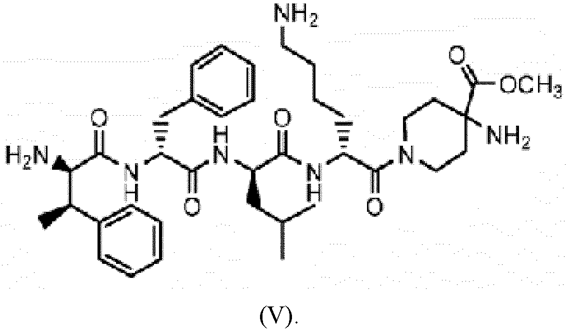

In some embodiments, the compound of formula (I) is: 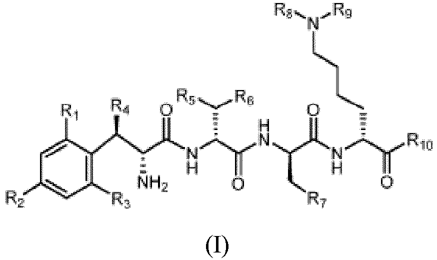 wherein:
R₁, R₂, and R₃ are independently selected from the group consisting of H, CN, Cl, F, unsubstituted C₁-C₈ alkyl, substituted C₁-C₈ alkyl, unsubstituted C₃-C₁₀ cycloalkyl, and substituted C₃-C₁₀ cycloalkyl;
R₄ is selected from H, unsubstituted C₁-C₈ alkyl, substituted C₁-C₈ alkyl, unsubstituted C₃-C₁₀ cycloalkyl, and substituted C₃-C₁₀ cycloalkyl;
R₅ and R₆ are independently selected from H, unsubstituted C₁-C₈ alkyl, substituted C₁-C₈ alkyl, unsubstituted C₃-C₁₀ cycloalkyl, substituted C₃-C₁₀ cycloalkyl, unsubstituted aryl, substituted aryl, unsubstituted heterocyclyl, and substituted heterocyclyl;
R₇ is selected from H, unsubstituted C₁-C₈ alkyl, substituted C₁-C₈ alkyl, unsubstituted C₃-C₁₀ cycloalkyl, and substituted C₃-C₁₀ cycloalkyl;
R₈ and R₉ are independently selected from H, unsubstituted C₁-C₈ alkyl, substituted C₁-C₈ alkyl, unsubstituted C₃-C₁₀ cycloalkyl, O-substituted C₁-C₈ alkyl, O-unsubstituted C₁-C₈ alkyl, CH₃O(CH₂CH₂)ₙCH₂CH₂-, and HO(CH₂CH₂)ₙCH₂CH₂-;
R₁₀ is 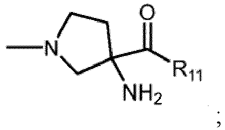
R₁₁ is OR₁₂ or NR₁₃R₁₄;
R₁₂ is selected from H, unsubstituted C₁-C₂₄ alkyl, substituted C₁-C₂₄ alkyl, O-substituted C₁-C₂₄ alkyl, O-unsubstituted C₁-C₂₄ alkyl, CH₃O(CH₂CH₂)ₙCH₂CH₂-, and HO(CH₂CH₂)ₙCH₂CH₂-;
R₁₃ and R₁₄ are independently selected from H, unsubstituted C₁-C₂₄ alkyl, substituted C₁-C₂₄ alkyl, O-substituted C₁-C₂₄ alkyl, O-unsubstituted C₁-C₂₄ alkyl, CH₃O(CH₂CH₂)ₙCH₂CH₂-, and HO(CH₂CH₂)ₙCH₂CH₂-; and
n is an integer from 0 to 100.

In some embodiments, in the compound of formula (I), wherein, R₁, R₂, and R₃ are independently selected from H, CN, Cl, F, unsubstituted C₁-C₄ alkyl, substituted C₁-C₄ alkyl, unsubstituted C₃-C₈ cycloalkyl, and substituted C₃-C₈ cycloalkyl;
R₄ is selected from unsubstituted C₁-C₄ alkyl, substituted C₁-C₄ alkyl, unsubstituted C₃-C₈ cycloalkyl, and substituted C₃-C₈ cycloalkyl;
R₅ and R₆ are independently selected from H, unsubstituted C₁-C₄ alkyl, substituted C₁-C₄ alkyl, unsubstituted C₃-C₈ cycloalkyl, substituted C₃-C₈ cycloalkyl, unsubstituted aryl, substituted aryl, unsubstituted heterocyclyl, and substituted heterocyclyl;
R₇ is selected from unsubstituted C₁-C₄ alkyl, substituted C₁-C₄ alkyl, unsubstituted C₃-C₈ cycloalkyl, and substituted C₃-C₈ cycloalkyl;
R₈ and R₉ are independently selected from H, unsubstituted C₁-C₄ alkyl, substituted C₁-C₄ alkyl, O-substituted C₁-C₄ alkyl, O-unsubstituted C₁-C₄ alkyl, CH₃O(CH₂CH₂)ₙCH₂CH₂-, and HO(CH₂CH₂)ₙCH₂CH₂-;
R₁₀ is 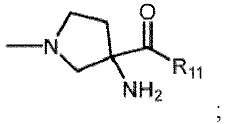
R₁₁ is selected from OR₁₂ and NR₁₃R₁₄;
R₁₂ is selected from H, unsubstituted C₁-C₁₂ alkyl, substituted C₁-C₁₂ alkyl, O-substituted C₁-C₁₂ alkyl, O-unsubstituted C₁-C₁₂ alkyl, CH₃O(CH₂CH₂)ₙCH₂CH₂-, and HO(CH₂CH₂)ₙCH₂CH₂-;
R₁₃ and R₁₄ are independently selected from H, unsubstituted C₁-C₁₂ alkyl, substituted C₁-C₁₂ alkyl, O-substituted C₁-C₁₂ alkyl, O-unsubstituted C₁-C₁₂ alkyl, CH₃O(CH₂CH₂)ₙCH₂CH₂-, and HO(CH₂CH₂)ₙCH₂CH₂-; and
n is an integer from 0 to 50.

In some embodiments, in the compound of formula (I), wherein, R₁, R₂, and R₃ are independently selected from H, Cl, F, methyl, ethyl, propyl, and isopropyl;
R₄ and R₇ are independently selected from methyl, ethyl, propyl, isopropyl, cyclopropyl, cyclobutyl, cyclopentyl, and cyclohexyl;
R₅ and R₆ are independently selected from H, methyl, ethyl, n-propyl, isopropyl, cyclopropyl, cyclobutyl, cyclopentyl, cyclohexyl, and phenyl;
R₈ and R₉ are independently selected from H, methyl, ethyl, propyl, and isopropyl;
R₁₀ is 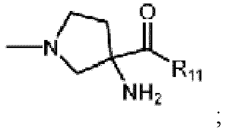
R₁₁ is selected from OR₁₂ and NR₁₃R₁₄;
R₁₂ is selected from H, methyl, ethyl, n-propyl, and isopropyl; and
R₁₃ and R₁₄ are independently selected from H, methyl, ethyl, n-propyl, isopropyl, cyclopropyl, cyclobutyl, cyclopentyl, and cyclohexyl.

In some embodiments, in the compound of formula (I), wherein, R₁, R₂, R₃, R₅, R₈, and R₉ are H; R₄ is methyl; R₆ is phenyl; R₇ is isopropyl;
R₁₀ is 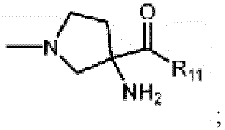
R₁₁ is OR₁₂; R₁₂ is H; and R₁₃, R₁₄, and n are absent, and the compound of formula (I) is a compound as represented by formula (II):

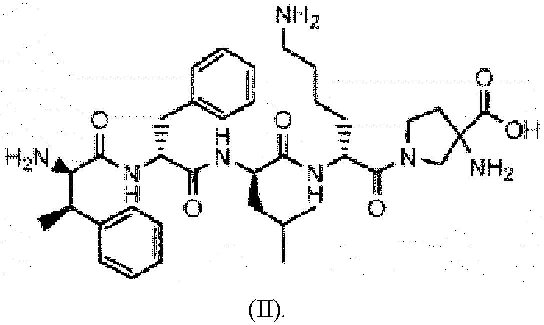

In some embodiments, in the compound of formula (I), wherein, R₁, R₂, R₃, R₃, R₈, and R₉ are H; R₄ is methyl; R₆ is phenyl; R₇ is isopropyl;
R₁₀ is 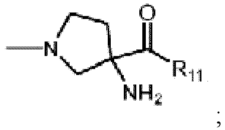
R₁₁ is OR₁₂; R₁₂ is Me; and R₁₃, R₁₄, and n are absent, the compound of formula (I) is a compound as represented by formula (III):

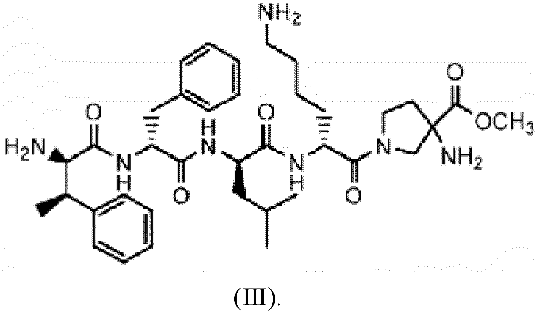

In some embodiments, the pharmaceutically acceptable salt is an acid addition salt. In some preferred embodiments, the pharmaceutically acceptable salt is selected from one of acetate, hydrobromide, phosphate, sulfate, formate, citrate, mesylate, maleate, malate, succinate, and fumarate. In some preferred embodiments, the pharmaceutically acceptable salt is an acetate salt.

In some preferred embodiments, the pharmaceutical composition comprises an acetate salt of a compound represented by formula (II), a pH adjusting agent, and water for injection. The pH adjusting agent is hydrochloric acid; and pH of the pharmaceutical composition is 3.0-5.0:

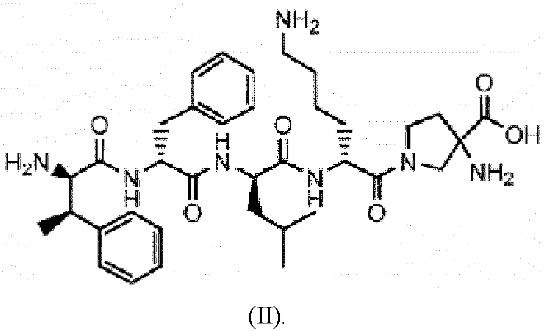

In some embodiments, the present application provides a pharmaceutical composition of a kappa opioid receptor agonist, which comprises an acetate salt of a compound represented by formula (II), a pH adjusting agent, and water for injection. The pH adjusting agent is hydrochloric acid; and pH of the pharmaceutical composition is 3.0-5.0.

In some embodiments, the present application provides a pharmaceutical composition of a kappa opioid receptor agonist, which comprises an acetate salt of a compound represented by formula (II), an osmotic pressure regulator, a pH adjusting agent, and water for injection. The pH adjusting agent is hydrochloric acid; and pH of the pharmaceutical composition is 3.0-5.0.

In some preferred embodiments, content of the pH adjusting agent is such that the pH of the pharmaceutical composition is 3.0-4.5, 3.5-5.0, or 3.5-4.5, alternatively, 4.0.

In some preferred embodiments, concentration of the pH adjusting agent is 0.01 mol/L-0.3 mol/L, preferably 0.1 mol/L.

In some preferred embodiments, content of the active ingredient is 0.01 mg/mL-50 mg/mL, such as 0.01 mg/mL-0.1 mg/mL, 0.1 mg/mL-0.2 mg/mL, 0.2 mg/mL-50 mg/mL. In some preferred embodiments, content of the active ingredient is 0.01 mg/mL, 0.1 mg/mL, 0.2 mg/mL or 50 mg/mL, preferably 0.1 mg/mL or 0.2 mg/mL.

In some preferred embodiments, the osmotic pressure regulator is selected from one or more of sodium chloride, mannitol, sorbitol, and glucose. In some preferred embodiments, the osmotic pressure regulator is sodium chloride.

In some preferred embodiments, content of the osmotic pressure regulator is 0.25 mg/mL-9 mg/mL, preferably 9 mg/mL.

In some preferred embodiments, the osmotic pressure regulator is used in an amount such that osmotic pressure of the pharmaceutical composition is 285 mOsmol/kg-310 mOsmol/kg.

In some preferred embodiments, the pharmaceutical composition is an injection solution.

In some preferred embodiments, the pharmaceutical composition is a lyophilized injection.

In some preferred embodiments, the pharmaceutical composition is an injection solution consisting of the following ingredients:

| | |
|---|---|
| an acetate salt of a compound of formula (II) | 0.01 mg/mL-50 mg/mL; |

hydrochloric acid used in an amount such that pH of the injection solution is 3.0-5.0;
and the balance of water for injection.

In some preferred embodiments, the pharmaceutical composition is an injection solution consisting of the following ingredients:

| | |
|---|---|
| an acetate salt of a compound of formula (II) | 0.01 mg/mL-50 mg/mL; |

hydrochloric acid used in an amount such that pH of the injection solution is 3.5-4.5;
and the balance of water for injection.

In some preferred embodiments, the pharmaceutical composition is an injection solution consisting of the following ingredients:

| | |
|---|---|
| an acetate salt of a compound of formula (II) | 0.1 mg/mL-0.2 mg/mL; |

hydrochloric acid used in an amount such that pH of the injection solution is 3.5-4.5;
and the balance of water for injection.

In some preferred embodiments, the pharmaceutical composition is an injection solution consisting of:

| | |
|---|---|
| an acetate salt of a compound of formula (II) | 0.1 mg/mL; |

hydrochloric acid used in an amount such that pH of the injection solution is 3.5-4.5;
and the balance of water for injection.

In some preferred embodiments, the pharmaceutical composition is an injection solution consisting of the following ingredients:

| | |
|---|---|
| an acetate salt of a compound of formula (II) | 0.2 mg/mL; |

hydrochloric acid used in an amount such that pH of the injection solution is 3.5-4.5;
and the balance of water for injection.

In some preferred embodiments, the pharmaceutical composition is an injection solution consisting of the following ingredients:

| | |
|---|---|
| an acetate salt of a compound of formula (II) | 0.1 mg/mL; |

hydrochloric acid used in an amount such that pH of the injection solution is 4.0;
and the balance of water for injection.

In some preferred embodiments, the pharmaceutical composition is an injection solution consisting of the following ingredients:

| | |
|---|---|
| an acetate salt of a compound of formula (II) | 0.2 mg/mL; |

hydrochloric acid used in an amount such that pH of the injection solution is 4.0;
and the balance of water for injection.

In some preferred embodiments, the pharmaceutical composition is an injection solution consisting of the following ingredients:

| | |
|---|---|
| an acetate salt of a compound of formula (II) | 0.01 mg/mL-50 mg/mL; |

hydrochloric acid used in an amount such that pH of the injection solution is 3.0-5.0;

| | |
|---|---|
| osmotic pressure regulator | 0.25 mg/mL-9 mg/mL; |

and the balance of water for injection;
wherein the osmotic pressure regulator is selected from one or more of sodium chloride, mannitol, sorbitol, and glucose.

In some preferred embodiments, the pharmaceutical composition is an injection solution consisting of the following ingredients:

| | |
|---|---|
| an acetate salt of a compound of formula (II) | 0.01 mg/mL-50 mg/mL; |

hydrochloric acid used in an amount such that pH of the injection solution is 3.5-4.5;

| | |
|---|---|
| osmotic pressure regulator | 0.25 mg/mL-9 mg/mL; |

and the balance of water for injection;
wherein the osmotic pressure regulator is selected from one or more of sodium chloride, mannitol, sorbitol, and glucose.

In some preferred embodiments, the pharmaceutical composition is an injection solution consisting of the following ingredients:

| | |
|---|---|
| an acetate salt of a compound of formula (II) | 0.1 mg/mL-0.2 mg/mL; |

hydrochloric acid used in an amount such that pH of the injection solution is 3.5-4.5;

| | |
|---|---|
| osmotic pressure regulator | 0.25 mg/mL-9 mg/mL; |

and the balance of water for injection; wherein the osmotic pressure regulator is selected from one or more of sodium chloride, mannitol, sorbitol, and glucose.

In some preferred embodiments, the pharmaceutical composition is an injection solution consisting of the following ingredients:

| | |
|---|---|
| an acetate salt of a compound of formula (II) | 0.1 mg/mL |

hydrochloric acid used in an amount such that pH of the injection solution is 3.5-4.5;
sodium chloride 9 mg/mL;
and the balance of water for injection.

In some preferred embodiments, the pharmaceutical composition is an injection solution consisting of the following ingredients:

| | |
|---|---|
| an acetate salt of a compound of formula (II) | 0.2 mg/mL; |

hydrochloric acid used in an amount such that pH of the injection solution is 3.5-4.5;
sodium chloride 9 mg/mL;
and the balance of water for injection.

In some preferred embodiments, the pharmaceutical composition is an injection solution consisting of the following ingredients:

| | |
|---|---|
| an acetate salt of a compound of formula (II) | 0.1 mg/mL; |

hydrochloric acid used in an amount such that pH of the injection solution is 4.0;
sodium chloride 9 mg/mL;
and the balance of water for injection.

In some preferred embodiments, the pharmaceutical composition is an injection solution consisting of the following ingredients:

| | |
|---|---|
| an acetate salt of a compound of formula (II) | 0.2 mg/mL; |

hydrochloric acid used in an amount such that pH of the injection solution is 4.0;
sodium chloride 9 mg/mL;
and the balance of water for injection.

In another aspect, the present application provides a method for preparing a pharmaceutical composition of a kappa opioid receptor agonist, which comprises:
dissolving an optional osmotic pressure regulator in water for injection to obtain Solution 1;
mixing the Solution 1 with an active ingredient to obtain Solution 2;
adjusting pH of the Solution 2 to 3.0-5.0 with hydrochloric acid solution to obtain Solution 3; and
making the Solution 3 up to volumn, filtering, filling and sealing, and sterilizing, to obtain the pharmaceutical composition.

In some preferred embodiments, pH of the Solution 2 is adjusted to 3.0-4.5, 3.5-5.0, 3.5-4.5, e.g. 4.0 with hydrochloric acid solution.

In some preferred embodiments, concentration of the aqueous hydrochloric acid solution is 0.01 mol/L-0.3 mol/L, preferably 0.1 mol/L.

In some preferred embodiments, the filtering in the aforementioned method for preparing the pharmaceutical composition refers to microporous membrane filtration.

In some preferred embodiments, the filling and sealing in the aforementioned method for preparing the pharmaceutical composition refers to purging with nitrogen, filling into an ampoule bottle, and sealing.

In some preferred embodiments, the sterilizing in the aforementioned method for preparing the pharmaceutical composition refers to high-temperature sterilizating, with the sterilization temperature of 121 °C and the sterilization time of 15 minutes.

In a third aspect, the present application provides a kappa opioid receptor agonist which is an acetate salt of a compound as represented by formula (II):

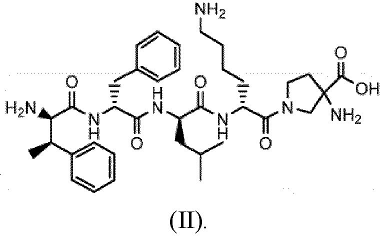

In a fourth aspect, the present application provides the aforementioned pharmaceutical composition of a kappa opioid receptor agonist or the acetate salt of the compound represented by formula (II) for use in preventing, treating, or treating and preventing a disease associated with a kappa opioid-like substance receptor.

In a fifth aspect, the present application provides use of the aforementioned pharmaceutical composition of a kappa opioid receptor agonist or the acetate salt of the compound represented by formula (II) in the preparation of a medicament for preventing, treating, or treating and preventing a disease associated with a kappa opioid-like substance receptor.

In a sixth aspect, the present application provides a method of preventing, treating, or treating and preventing a disease associated with a kappa opioid-like substance receptor, which comprises administering to an individual in need thereof a therapeutically effective amount of the aforementioned pharmaceutical composition of a kappa opioid receptor agonist or the acetate salt of the compound represented by formula (II).

In some embodiments of the fourth or fifth or sixth aspect, the disease associated with a kappa opioid-like substance receptor is selected from one or more of pain, cardiovascular disease, pruritus, nausea, inflammation, spinal anesthesia, cough, stroke, hypoxic pulmonary hypertension, multiple sclerosis, addiction, and post-traumatic cartilage degeneration.

The present application provides a pharmaceutical composition of a kappa opioid receptor agonist, a preparation method therefor, and use thereof. The pharmaceutical composition of the present application uses hydrochloric acid as a pH adjusting agent, greatly reducing degradation impurities produced by the active substance in the present application during high-temperature sterilization, and increasing the stability of the pharmaceutical composition. In addition, the pharmaceutical compositions, formulations and processes of the present application facilitate commercial production. The prescription is simple and compatible with clinical use, and the stability of the pharmaceutical composition of the kappa opioid receptor agonist can be guaranteed in production and use.

In this application, unless otherwise specified, scientific and technical terms used herein have the meanings commonly understood by those skilled in the art.

### Detailed Description

To further clarify the purpose, technical solutions and advantages of the present application, examples of the present application will be described in detail below. The following examples are intended to illustrate the present invention only and should not be considered as limiting the scope of the present application. Where specific conditions are not indicated in the examples, conventional conditions or conditions recommended by the manufacturer shall be followed. The reagents or instruments used without indication of the manufacturer are all conventional products that are commercially available.

### Example 1

### Preparation of acetate salt of compound of formula (II)

290 g of acetyl chloride and 370 g of anhydrous methanol were used to formulate hydrochloric acid-methanol solution. 52 g of methyl 1-(N⁶-(tert-butoxycarbonyl)-N²-((2R, 3R)-2-((tert-butoxycarbonyl)amino)-3-phenylbutanoyl)-D-phenylalanyl-D-leucyl-D-lysyl)-3-((t ert-butoxycarbonyl)amino)pyrrolidine-3-carboxylate (which is prepared with reference to the synthetic method of peptide analog 12 in Example 7 of WO2021262173A1) was added. Reaction was performed at 40 °C or lower until the reaction was completed. After the reaction was completed, it was concentrated until there were no droplets, dissolved in methanol, added with 1 M sodium hydroxide solution to hydrolyze completely, and the pH was adjusted to 5 with acetic acid. After the reaction solution was concentrated, a system of 0.1% aqueous acetic acid solution and acetonitrile was used for preparation and purification, and freeze-dried to obtain 29.5 g of acetate salt of the compound of formula (II) with a yield of 76% and a purity of 99.1%.

The NMR characterization was as follows:
¹H NMR (500 MHz, D₂O): δ 0.91 (d, 3H), 0.96 (m, 3H), 1.33 (m, 3H), 1.49 (m, 2H), 1.58 (m, 1H), 1.59 (m, 2H), 1.76 (m, 2H), 1.81 (m, 2H), 1.95 (s, 3H), 2.31~2.70 (m, 2H), 3.05 (m, 2H), 3.12 (m, 2H), 3.21 (m, 1H), 3.78&4.00 (m, 2H), 3.79&3.97 (m, 1H), 4.07&4.18 (m, 1H), 4.04 (m, 1H), 4.43 (m, 2H), 4.78 (m, 1H), 7.3 (m, 2H), 7.34 (m, 1H), 7.35 (m, 2H), 7.38 (m, 1H), 7.4 (m, 2H), 7.46 (m, 2H).
¹³C NMR (125MHz, D₂O): δ 17.25, 21.14, 22.23, 22.03, 23.38, 24.23, 26.53, 29.77, 32.57~34.63, 37.17, 39.22, 40.03, 41.77, 45.02~45.51, 51.31~51.98, 53.54~54.18, 54.88, 58.75, 62.88~65.45, 127.22, 127.88, 128.2, 128.82, 129.23, 129.35, 136.05, 139.75, 169.56, 171.73, 173.11~173.44, 173.85, 181.04.
LC/MS (ESI+) m/z 680 [M-CH₃COOH+H]⁺.

### Example 2

### Preparation of citrate salt of compound of formula (II)

The acetate salt of the compound of formula (II) (300 mg, 0.41 mmol) was added to 12 ml of water at room temperature, stirred to dissolve, added with 1 M sodium hydroxide solution to adjust the pH to 9, then added with 1 M aqueous citric acid solution to adjust the pH to 4-5, and stirred at room temperature for 1 h. After concentration, a water and acetonitrile system was used in liquid phase for preparation and purification, then concentrated, and freeze-dried to obtain the target product (259 mg, yield 72%). The ion chromatographic detection results of the obtained product showed that the content of the citrate ion was 22.0%, indicating that the molar ratio of the main ingredient to citric acid in the salt was 1:1.

### Example 3

### Preparation of phosphate salt of compound of formula (II)

The acetate salt of the compound of formula (II) (200 mg, 0.27 mmol) was added to 8 ml of water at room temperature, stirred to dissolve, added with 1 M sodium hydroxide solution to adjust the pH to 9, then added with 1 M aqueous phosphoric acid solution to adjust the pH to 4-5, and stirred at room temperature for 1 h. After concentration, a water and acetonitrile system was used in liquid phase for preparation and purification, then concentrated, and freeze-dried to obtain the target product (172 mg, yield 82%). The phosphate ion content of the obtained product was 12.6% by ion chromatography, indicating that the molar ratio of the main ingredient to phosphoric acid in the salt was 1:1.

### Example 4

### Investigation on the stability of salts of compound of formula (II)

Samples of acetate salt, citrate salt, and phosphate salt of the compound of formula (II) were placed for investigation respectively, and the investigation conditions were high temperature test (40 °C) and light (4500 Lux). The investigation period was 5 days, 10 days and 30 days. The stability results were shown in Table 1 below:

**Table 1. Investigation results of stability of different salts of compound of formula (II)**

| Condition | Time/Day | Acetate salt (1:1) HPLC purity (%) | Citrate salt (1:1) HPLC purity (%) | Phosphate salt (1:1) HPLC purity (%) |
|---|---|---|---|---|
| 40 °C | 0 days | 97.9% | 96.8% | 96.5% |
| | 5 days | 97.7% | 95.7% | 95.6% |
| | 10 days | 97.5% | 94.6% | 94.3% |
| | 30 days | 97.1% | 93.5% | 92.3% |
| 4500 Lux | 5 days | 97.9% | 96.2% | 96.2% |
| | 10 days | 97.8% | 95.5% | 95.1% |
| | 30 days | 97.6% | 94.7% | 94.2% |

The results showed that the citrate salt and phosphate salt of the compound of formula (II) were degraded in different degrees when investigated at high temperature of 40 °C and under light of 4500 Lux. The acetate salt showed better stability both in high temperature and under light.

### Example 5

### Influence of different pH adjusting agents on the stability of injection solutions

Water for injection with a formulation amount of about 80 g was added into a mixing tank, 10 mg of acetate salt of the compound of formula (II) (Example 1) was added under stirring, and stirring was continued until a uniformly mixed pharmaceutical solution was obtained. The pH value of the pharmaceutical solution was adjusted to about 4.5 (for specific values, referring to the pH values in Table 2 below) with a pH adjusting agent of 0.1 mol/L (the type of pH adjusting agent was shown in Table 2 below), and stirring was continued until uniformly mixed, obtaining a pH-adjusted pharmaceutical solution. Water for injection was added to the aforementioned pharmaceutical solution to make the volume up to 100 ml, then the solution was filtered and sterilized (121 °C, 15 minutes), and the properties and characteristics were observed and the total impurity content was determined. The results were shown in Table 2 below.

**Table 2. Investigation results of stability of injection solutions using different pH adjusting agents**

| Sample No. | pH adjusting agent | Finished product | | | 25 °C for one month | 25 °C for two months | 25 °C for three months |
|---|---|---|---|---|---|---|---|
| | | pH value | Product properties and characteristics after sterilization | Total impurities (%) | Total impurities (%) | Total impurities (%) | Total impurities (%) |
| 1 | Acetic acid-sodium acetate | 4.6 | Producing flocculent precipitate | 1.3 | 1.5 | 1.7 | 2.0 |
| 2 | Hydrochloric acid | 4.6 | Clear colorless liquid | 0.24 | 0.21 | 0.23 | 0.22 |
| 3 | Citric acid-sodium citrate | 4.8 | Producing flocculent precipitate | 1.0 | 1.2 | 1.3 | 1.5 |
| 4 | Phosphoric acid-disodium hydrogen phosphate | 4.8 | Producing flocculent precipitate | 1.4 | 1.5 | 1.7 | 1.9 |
| 5 | Sulfuric acid | 4.7 | Clear colorless liquid | 0.53 | 0.71 | 1.1 | 1.4 |
| 6 | Sodium bisulfate | 4.6 | Producing flocculent precipitate | 1.4 | 1.8 | 2.1 | 2.3 |
| 7 | Lactic acid | 4.8 | Producing flocculent precipitate | 1.7 | 2.0 | 2.4 | 2.7 |

From the results in the above table, it could be seen that when acetic acid-sodium acetate, citric acid-sodium citrate, phosphoric acid-disodium hydrogen phosphate, sodium bisulfate and lactic acid were used as the pH adjusting agent, the aforementioned experimental pharmaceutical solutions, after being sterilized at high temperature, all produced white flocculent precipitate, which was determined, after detection, to be six-membered ring degradation impurities, and the total impurity content was higher than 1%. When hydrochloric acid or sulfuric acid was used as the pH adjusting agent, the pharmaceutical solution was still a clear colorless liquid after high-temperature sterilization. There was no flocculent precipitate of degradation impurities and the total impurity content was relatively low. Especially, the total impurity content in the pharmaceutical solution was only 0.2% after sterilization at 121 °C for 15 min in the hydrochloric acid system. Moreover, during the long-term investigation in the later period, the impurities in the injection solutions composed of other pH adjusting agents increased significantly, but in the hydrochloric acid system, there was basically no increase in the impurities.

### Example 6

### Influence of concentration of hydrochloric acid on stability of injection solutions

Water for injection with a formulation amount of about 80 g was added into a mixing tank, 10 mg of acetate salt of the compound of formula (II) (Example 1) was added under stirring, and stirring was continued until a uniformly mixed pharmaceutical solution was obtained. The pH value of the pharmaceutical solution was adjusted to about 4.5 (for specific values, referring to the pH values in Table 3 below) with hydrochloric acid (the concentration of hydrochloric acid was shown in Table 3 below), and stirring was continued until uniformly mixed, obtaining a pH-adjusted pharmaceutical solution. Water for injection was added to the aforementioned pharmaceutical solution to make the volume up to 100 ml, then the solution was filtered and sterilized (121 °C, 15 minutes) to obtain finished products. The finished products corresponding to each concentration were placed at 60 °C for 5 days, 10 days, and 30 days respectively, and their properties and characteristics and total impurity content were detected. The results were shown in Table 3 below.

**Table 3. Stability investigation results of injection solutions using different concentrations of hydrochloric acid**

| Sample | Concentration of pH adjusting agent | pH value | Product properties and characteristics after sterilization | Total impurities% |
|---|---|---|---|---|
| Finished product | 0.01 mol/L | 4.5 | Clear colorless liquid | 0.25 |
| | 0.1 mol/L | 4.6 | Clear colorless liquid | 0.21 |
| | 0.2 mol/L | 4.5 | Clear colorless liquid | 0.23 |
| | 0.3 mol/L | 4.5 | Clear colorless liquid | 0.21 |
| 60 °C for 5 days | 0.01 mol/L | 4.6 | Clear colorless liquid | 0.26 |
| | 0.1 mol/L | 4.5 | Clear colorless liquid | 0.21 |
| | 0.2 mol/L | 4.6 | Clear colorless liquid | 0.25 |
| | 0.3 mol/L | 4.5 | Clear colorless liquid | 0.22 |
| 60 °C for 10 days | 0.01 mol/L | 4.5 | Clear colorless liquid | 0.27 |
| | 0.1 mol/L | 4.6 | Clear colorless liquid | 0.23 |
| | 0.2 mol/L | 4.5 | Clear colorless liquid | 0.26 |
| | 0.3 mol/L | 4.5 | Clear colorless liquid | 0.22 |
| 60 °C for 30 days | 0.01 mol/L | 4.7 | Clear colorless liquid | 0.45 |
| | 0.1 mol/L | 4.6 | Clear colorless liquid | 0.42 |
| | 0.2 mol/L | 4.6 | Clear colorless liquid | 0.43 |
| | 0.3 mol/L | 4.7 | Clear colorless liquid | 0.42 |

From the test results in Table 3 above, it could be seen that when the concentration of hydrochloric acid for adjusting pH was within the concentration range of 0.01 mol/L to 0.3 mol/L, all of the injection solutions composed of acetate salts of compound of formula (II) could maintain good stability.

### Example 7

Influence of pH ranges adjusted by hydrochloric acid on stability of inj ection solutions Water for inj ection with a formulation amount of about 80 g was added into a mixing tank, 10 mg of acetate salt of the compound of formula (II) (Example 1) was added under stirring, and stirring was continued until a uniformly mixed pharmaceutical solution was obtained. The pH value of the pharmaceutical solution was adjusted (for specific pH values, referring to the pH values in Table 4 below) with 0.1 mol/L hydrochloric acid, and stirring was continued until uniformly mixed, obtaining a pH-adjusted pharmaceutical solution. Water for injection was added to the aforementioned pharmaceutical solution to make the volume up to 100 ml, then the solution was filtered and sterilized (121 °C, 15 minutes) to obtain finished products. The finished products corresponding to each pH value were placed at 60 °C for 5 days, 10 days, and 30 days respectively, and their properties and characteristics and total impurity content were detected. The results were shown in Table 4 below.

**Table 4. Stability investigation results of in ection solutions with different pH values**

| **Sample** | **Properties and characteristics** | **Solution clarity and color** | **Visible foreign bodies** | **pH value** | **Total impurities %** |
|---|---|---|---|---|---|
| 0 days | Colorless clear liquid | Clear colorless liquid | Meet the requirements | 2.5 | 0.36 |
| | Colorless clear liquid | Clear colorless liquid | Meet the requirements | 3.1 | 0.21 |
| | Colorless clear liquid | Clear colorless liquid | Meet the requirements | 3.5 | 0.21 |
| | Colorless clear liquid | Clear colorless liquid | Meet the requirements | 4.0 | 0.23 |
| | Colorless clear liquid | Clear colorless liquid | Meet the requirements | 4.6 | 0.24 |
| | Colorless clear liquid | Clear colorless liquid | Meet the requirements | 5.0 | 0.24 |
| High temperature for 5 days | Colorless clear liquid | Clear colorless liquid | Meet the requirements | 2.6 | 0.41 |
| | Colorless clear liquid | Clear colorless liquid | Meet the requirements | 3.1 | 0.30 |
| | Colorless clear liquid | Clear colorless liquid | Meet the requirements | 3.5 | 0.28 |
| | Colorless clear liquid | Clear colorless liquid | Meet the requirements | 4.1 | 0.25 |
| | Colorless clear liquid | Clear colorless liquid | Meet the requirements | 4.6 | 0.27 |
| | Colorless clear liquid | Clear colorless liquid | Meet the requirements | 5.2 | 0.28 |
| High temperature for 10 days | Colorless clear liquid | Clear colorless liquid | Meet the requirements | 2.6 | 0.43 |
| | Colorless clear liquid | Clear colorless liquid | Meet the requirements | 3.1 | 0.37 |
| | Colorless clear liquid | Clear colorless liquid | Meet the requirements | 3.6 | 0.35 |
| | Colorless clear liquid | Clear colorless liquid | Meet the requirements | 4.2 | 0.32 |
| | Colorless clear liquid | Clear colorless liquid | Meet the requirements | 4.7 | 0.34 |
| | Colorless clear liquid | Clear colorless liquid | Meet the requirements | 5.1 | 0.35 |
| High temperature for 30 days | Colorless clear liquid | Clear colorless liquid | Meet the requirements | 2.6 | 0.58 |
| | Colorless clear liquid | Clear colorless liquid | Meet the requirements | 3.1 | 0.45 |
| | Colorless clear liquid | Clear colorless liquid | Meet the requirements | 3.6 | 0.43 |
| | Colorless clear liquid | Clear colorless liquid | Meet the requirements | 4.1 | 0.41 |
| | Colorless clear liquid | Clear colorless liquid | Meet the requirements | 4.6 | 0.46 |
| | Colorless clear liquid | Clear colorless liquid | Meet the requirements | 5.1 | 0.45 |

From the test results in Table 4 above, it could be seen that when the pH value was around 2.5, the impurities in the injection solutions composed of acetate salts of compound of formula (II) increased rapidly; when the pH value was 3.0-5.0, the injection solutions composed of acetate salts of compound of formula(II) were all stable. Therefore, the pH range of this product was between 3.0 and 5.0, preferably the pH range was between 3.5 and 4.5, and more preferably 4.0.

### Example 8

Influence of different osmotic pressure regulators on the stability of injection solutions Water for injection with a formulation amount of about 80 g and an osmotic pressure regulator (specific types and dosages were shown in Table 5 below) were added into a mixing tank. After mixing uniformly, 10 mg of acetate salt of the compound of formula (II) (Example 1) was added under stirring, and stirring was continued until a uniformly mixed pharmaceutical solution was obtained. The pH value of the pharmaceutical solution was adjusted to about 4.0 (for specific values, referring to the pH values in Table 5 below) with 0.1 mol/L hydrochloric acid, and stirring was continued until uniformly mixed, obtaining a pH-adjusted pharmaceutical solution. Water for injection was added to the aforementioned pharmaceutical solution to make the volume up to 100 ml, then the solution was filtered and sterilized (121 °C, 15 minutes) to obtain finished products, which were detected for their properties and characteristics, osmotic pressure, and total impurity content. The results were shown in Table 5 below.

**Table 5. Investigation results of different osmotic pressure regulators on the stability of injection solutions**

| Osmotic pressure regul ator | NA | Mannitol | Sodium chloride | Glucose |
|---|---|---|---|---|
| Dosage | NA | 5.07% (W/V) | 0.9% (W/V) | 5% (W/V) |
| pH value | 4.0 | 4.1 | 4.1 | 4.0 |
| Solution clarity and color | Clear colorless liquid | Clear colorless liquid | Clear colorless liquid | Clear colorless liquid |
| Total impurities% | 0.25 | 0.23 | 0.26 | 0.24 |
| Osmotic pressure mOsmol/kg | 0 | 305 | 298 | 287 |

From the test results in Table 5 above, it could be seen that when sodium chloride, glucose or mannitol was used as the osmotic pressure regulator, osmotic pressures of the solutions were all within the range required by the pharmacopoeia. Sodium chloride is preferably used as the osmotic pressure regulator in terms of clinical safety and product stability.

### Example 9

### Influence of dosages of active ingredients on stability of injection solutions

Water for injection with a formulation amount of about 80 g and an osmotic pressure regulator (specific types and dosages were shown in Table 6 below) were added into a mixing tank. After mixing uniformly, acetate salt of the compound of formula (II) (Example 1) (for specific values, referring to the dosages in Table 6 below) was added under stirring and stirring was continued until a uniformly mixed pharmaceutical solution was obtained. The pH value of the pharmaceutical solution was adjusted to about 4.0 (for specific values, referring to the pH values in Table 6 below) with 0.1 mol/L hydrochloric acid, and stirring was continued until uniformly mixed, obtaining a pH-adjusted pharmaceutical solution. Water for injection was added to the aforementioned pharmaceutical solution to make the volume up to 100 ml, then the solution was filtered and sterilized (121 °C, 15 minutes) to obtain finished products, which were detected for their properties and characteristics, osmotic pressure, and total impurity content. The results were shown in Table 6 below.

**Table 6. Investigation results of active ingredient dosage on the stability of injection solutions**

| Prescribing information | Prescription 1 | Prescription 2 | Prescription 3 | Prescription 4 | Prescription 5 |
|---|---|---|---|---|---|
| Dosage of active ingredient (mg) | 1 | 10 | 20 | 3000 | 5000 |
| Osmotic pressure regulator | Glucose | Sodium chloride | Sodium chloride | Sodium chloride | -- |
| Dosage of osmotic pressure regulator (g) | 5 | 0.9 | 0.9 | 0.45 | -- |
| pH value | 4.0 | 4.0 | 4.0 | 4.0 | 4.0 |
| Formulation amount (ml) | 100 | 100 | 100 | 100 | 100 |
| Concentration of main drug | 0.01 mg/ml | 0.1 mg/ml | 0.2 mg/ml | 30 mg/ml | 50 mg/ml |
| Solution clarity and color | Clear colorless liquid | Clear colorless liquid | Clear colorless liquid | Clear colorless liquid | Clear colorless liquid |
| Osmotic pressure mOsmol/kg | 306 | 305 | 306 | 312 | 293 |
| Total impurities% | 0.25 | 0.23 | 0.24 | 0.22 | 0.24 |

| | | | | | |
|---|---|---|---|---|---|
| Conclusion: From the above test results, it could be seen that when the concentration of main drug was 0.01 mg/ml to 50 mg/ml, within the pH range of Example 7, prescriptions of all concentrations were stable without significant difference. | | | | | |

### Example 10

An influencing factor test investigation and an accelerated test study were performed on the injection solution prepared according to Prescription 2 of Example 9 with reference to the drug stability guideline ICH Q1a.

Influencing factor test: high temperature test, with the temperature of 60 °C. The results were shown in Table 7 below. It could be seen from Table 7 that there was no significant change in various indexes in the injection solution after placing at a temperature of 60 °C for 30 days, indicating that this product had good stability.

**Table 7. Results of high temperature test**

| Time of investigat ion | Properties and characteris tics | Solution clarity and color | Visible foreign bodies | pH | Osmotic pressure mOsmol/k g | Insoluble particles | Total impuritie s% | Conten t %* |
|---|---|---|---|---|---|---|---|---|
| 0 days | Colorless clear solution | Meet the requireme nts | Meet the requireme nts | 4.0 | 304 | (3/0) | 0.21 | 100.1 |
| 5 days | Colorless clear solution | Meet the requireme nts | Meet the requireme nts | 4.1 | 305 | (7/1) | 0.27 | 100.2 |
| 10 days | Colorless clear solution | Meet the requireme nts | Meet the requireme nts | 4.1 | 308 | (0/0) | 0.32 | 100.1 |
| 30 days | Colorless clear solution | Meet the requireme nts | Meet the requireme nts | 4.2 | 305 | (0/7) | 0.42 | 100.3 |

| | | | | | | | | |
|---|---|---|---|---|---|---|---|---|
| * the content determined by HPLC, the same below. | | | | | | | | |

Influencing factor test: light test, with the light conditions of: total illumination ≥ 1.2 × 106 Lux·hr, near ultraviolet energy ≥ 200 w·hr/m². The results were shown in Table 8 below. It could be seen from Table 8 that there were no significant changes in various indexes in the injection solution after placing under light for 30 days, indicating that this product had good stability.

**Table 8. Results of light test**

| Time of investiga tion | Properties and characteristics | Solution clarity and color | Visible foreign bodies | pH | Osmotic pressure mOsmol /kg | Insolu ble particl es | Total impuriti es% | Cont ent % |
|---|---|---|---|---|---|---|---|---|
| 0 days | Colorless clear solution | Meet the requirements | Meet the requirements | 4.0 | 304 | (3/0) | 0.21 | 100.1 |
| 5 days | Colorless clear solution | Meet the requirements | Meet the requirements | 4.1 | 305 | (7/0) | 0.21 | 100.1 |
| 10 days | Colorless clear solution | Meet the requirements | Meet the requirements | 4.1 | 305 | (6/0) | 0.22 | 100.3 |
| 30 days | Colorless clear solution | Meet the requirements | Meet the requirements | 4.0 | 305 | (5/0) | 0.21 | 100.4 |

Influencing factor test: freeze-thaw experiment, the specific conditions were: three freeze-thaw cycles, conditions of each cycle being: placing at a temperature of -10 °C to -20 °C for 2 days, and then placing at 40 °C for 2 days. The results were shown in Table 9 below. It could be seen from Table 9 that after three cycles of freeze-thaw test, there was no significant change in various indexes in the injection solution, indicating that this product had good stability.

**Table 9. Results of freeze-thaw test**

| Time of investig ation | Properties and characteristics | Solution clarity and color | Visible foreign bodies | pH | Osmotic pressure mOsmol/ kg | Insolu ble particl es | Total impur ities% | Conten t% |
|---|---|---|---|---|---|---|---|---|
| 0 days | Colorless clear solution | Meet the requirements | Meet the requirements | 4.0 | 304 | (3/0) | 0.21 | 100.1 |
| Freeze-t haw once | Colorless clear solution | Meet the requirements | Meet the requirements | 4.1 | 302 | (20/3) | 0.23 | 100.2 |
| Freeze-t haw for 2 times | Colorless clear solution | Meet the requirements | Meet the requirements | 4.1 | 304 | (8/3) | 0.22 | 100.3 |
| Freeze-t haw for 3 times | Colorless clear solution | Meet the requirements | Meet the requirements | 4.0 | 305 | (8/0) | 0.21 | 100.4 |

Influencing factor test: accelerated test, an injection sample was prepared according to Prescription 2 in Example 9, and accelerated test study was performed on the sample. Under the market packaging conditions, the accelerated test was performed in a 40 °C stabilization box, and the results were shown in Table 10. From Table 10, it could be seen that there was no significant change in various indexes in the injection solution, indicating that the stability of this product was good.

**Table 10. Results of accelerated test**

| Time of investigat ion | Propertie s and characteri stics | Solution clarity and color | Visible foreign bodies | pH | Osmotic pressure mOsmol/kg | Insoluble particles | Total impuri ties% | Content % |
|---|---|---|---|---|---|---|---|---|
| 0 days | Colorless clear solution | Meet the requirement s | Meet the requireme nts | 4.0 | 304 | 3/0 | 0.21 | 100.1 |
| 1 month accelerati on | Colorless clear solution | Meet the requirement s | Meet the requireme nts | 4.0 | 305 | 5/0 | 0.22 | 100.3 |
| 2 month accelerati on | Colorless clear solution | Meet the requirement s | Meet the requireme nts | 4.1 | 306 | 10/2 | 0.24 | 100.2 |
| 3 month accelerati on | Colorless clear solution | Meet the requirement s | Meet the requireme nts | 4.1 | 304 | 18/3 | 0.25 | 100.1 |

The experimental results of the influencing factor test investigation and accelerated test study of the injection solution prepared according to the Prescription 3 of Example 9 were consistent with those of Example 10, and both had excellent stability. Furthermore, during the implementation of Examples, no precipitation occurred.

### Example 11 Analgesic effect test

### Experiment objective: To evaluate the analgesic activity of the composition of the present application using a rat postoperative analgesic model

Test sample: Prescription 5 of Example 9 of the present application was used as the test sample.

Sprague-Dawley rats, SPF grade, male, weighing 200 g-220 g, raised in a constant temperature environment, temperature (20±2) °C, humidity 40%-70%; the raising environment was with a light rhythm 12h: 12h (6:00-18:00), free feeding and water, and adaptive feeding for 1 week.

Animals were anesthetized with Zoletil (6 mg/kg) + xylazine (86 mg/kg, i.p.). The toes of the animals were squeezed to confirm that the animals were fully anesthetized prior to surgery. The skin of the surgical area on the plantar site of the left hind foot was disinfected three times using iodophor and 70% ethanol. Wait until the skin was dry, to start the surgery. An incision about 1 cm long was made longitudinally toward the toe from the heel position. After incising the skin, the flexor digitorum brevis was lifted and a longitudinal blunt injury was caused. After pressing to stop bleeding, the wound was sutured. After surgery, the animals were placed on electric blankets, and returned to the cage when the animals were fully awake (free to move).

Twenty-four hours after surgery, the baseline test of mechanical allodynia was performed on rats. The animals, after eliminating those that did not show mechanical allodynia, were randomly divided into four groups of 10 animals per group, which were the vehicle control group; morphine (positive drug) control group (4 mg/kg); test group 1 (0.1 mg/kg); test group 2 (0.5 mg/kg); and test group 3 (1.5 mg/kg). At the same time, 10 rats of the same batch were taken as the control of the normal group. Mechanical allodynia tests were performed at 0.5 h, 1 h, and 1.5 h after administration, respectively.

The rats were individually placed in a plexiglass box with a grid at the bottom to ensure that the rat feet could be tested. Rats would acclimatize for 15 minutes prior to the test. After completion of the acclimatizing, test fiber (Touch-Test Sensory Evaluator) was used to test at the central plantar site of the left hind foot of the rats. In the test, the test fiber was pressed vertically against the skin and force was applied to bend the fiber for 6-8 seconds, with 5 seconds apart between each test. In the test, an animal's rapid withdrawal of the foot was noted as a pain response. Mechanical allodynia was expressed as Paw Withdrawal Threshold (PWT) in rat behavioral test, and the equation for calculating the rat mechanical pain inhibition rate was: rat mechanical pain inhibition rate = (post-administration paw withdrawal threshold value - average paw withdrawal threshold value of vehicle group)/(average paw withdrawal threshold value of normal group - average paw withdrawal threshold value of vehicle group) × 100%. Calculated according to the equation, the results of mechanical pain inhibition rate in rats were shown in Table 11 below:

**Table 11. Pain inhibition rate of the composition of the present application in a rat postoperative analgesic model**

| Groups | Pain inhibition rate | | |
|---|---|---|---|
| | 0.5 hours | 1 hours | 1.5 hours |
| Morphine control group (3 mg/kg) | 122.5% | 84.2% | 27.9% |
| Test group 1 (0.1 mg/kg) | 10.3% | 7.4% | 2.5% |
| Test group 2 (0.5 mg/kg) | 69.0% | 35.3% | 11.4% |
| Test group 3 (1.5 mg/kg) | 99.0% | 55.3% | 32.3% |

From the data in Table 11, it could be seen that the composition of the present application at doses of 0.5 mg/kg and 1.5 mg/kg, compared with the vehicle group, significantly inhibited the mechanical allodynia induced by the postoperative analgesic model of rats, and had the dose-dependent analgesic activity in the dosing range of 0.5 mg/kg to 1.5 mg/kg. The analgesic effect at a dose of 1.5 mg/kg was comparable to that of morphine.

### Example 12 Antipruritic effect test

### Experiment objective: To evaluate the antipruritic effect of the composition of the present application using chloroquine-induced pruritus model of ICR mice

Test sample: Prescription 5 of Example 9 of the present application was used as the test sample.

### Experimental contents:

ICR mice, SPF grade, male, weighing 30 g-40 g, raised in a constant temperature environment, temperature (20 ± 2) °C, humidity 40%-70%; the raising environment was with a light rhythm 12h: 12h (6:00-18:00), free feeding and water, and adaptive feeding for 1 week.

The mice were shaved on the back of the neck. After shaving, the animals were weighed, and the mice were randomly divided into 5 groups of 10 animals per group according to their body weight, which were the vehicle control group; ICI204448 (CAS No.: 121264-04-8, positive control drug) control group (3 mg/kg); Test group 1 (0.1 mg/kg); Test group 2 (0.5 mg/kg); and Test group 3 (1.5 mg/kg). After the corresponding drugs were administered subcutaneously according to groups, 400 µg of chloroquine, a pruritus model inducer, was intradermally injected into the shaved area of the back of the neck of mice. Following intradermal injection of chloroquine, mice were individually placed in transparent plexiglass boxes and the number of scratches over 30 min was counted.

**Table 12. Pharmacodynamic evaluation of the compositions of the present application in chloroquine-induced pruritus model in mice**

| Groups | Number of scratches (mean ± standard error) | Inhibition rate |
|---|---|---|
| Vehicle control group | 283±26.86 | 0% |
| ICI204448 (3 mg/kg) | 7.3±2.43** | 97.4% |
| Test group 1 (0.1 mg/kg) | 185±30.24* | 34.6% |
| Test group 2 (0.5 mg/kg) | 105±10.56** | 62.9% |
| Test group 3 (1.5 mg/kg) | 5.6±1.76** | 98.0% |

| | | |
|---|---|---|
| Note: *p < 0.05, **p < 0.01 compared to vehicle group. | | |

From the data in Table 12, it could be seen that in the chloroquine-induced ICR mouse pruritus model, the composition of the present application could significantly inhibit the chloroquine-induced pruritus behavior response at a dose of 0.1 mg/kg or more, and its antipruritus effect was comparable to that of the positive control drug ICI204448 (3 mg/kg) at a dose of 1.5 mg/kg, and had dose-dependent antipruritus activity.

Although the embodiments disclosed in the present application are as above, the contents described are only the embodiments adopted for the convenience of understanding the present application, and are not used to limit the present application. Any person skilled in the art to which this application belongs may make any modifications and changes in the form and details of implementation without departing from the idea and scope disclosed in this application, but the scope of protection of this application shall still be subject to the scope defined in the appended claims.

## Claims

1. A pharmaceutical composition of a kappa opioid receptor agonist, the pharmaceutical composition comprising a compound represented by formula (I), or a stereoisomer thereof, or a metabolite thereof or a pharmaceutically acceptable salt thereof, optionally an osmotic pressure regulator, and a pH adjusting agent and water for injection;
wherein the compound represented by formula (I) is: 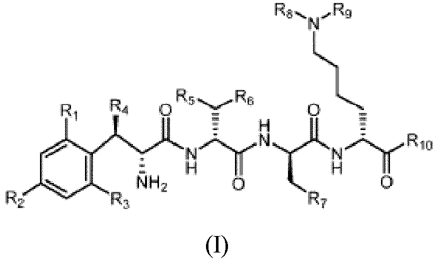
wherein:
R₁, R₂, and R₃ are independently selected from the group consisting of H, CN, Cl, F, unsubstituted C₁-C₈ alkyl, substituted C₁-C₈ alkyl, unsubstituted C₃-C₁₀ cycloalkyl, and substituted C₃-C₁₀ cycloalkyl;
R₄ is selected from H, unsubstituted C₁-C₈ alkyl, substituted C₁-C₈ alkyl, unsubstituted C₃-C₁₀ cycloalkyl, and substituted C₃-C₁₀ cycloalkyl;
R₅ and R₆ are independently selected from H, unsubstituted C₁-C₈ alkyl, substituted C₁-C₈ alkyl, unsubstituted C₃-C₁₀ cycloalkyl, substituted C₃-C₁₀ cycloalkyl, unsubstituted aryl, substituted aryl, unsubstituted heterocyclyl, and substituted heterocyclyl;
R₇ is selected from H, unsubstituted C₁-C₈ alkyl, substituted C₁-C₈ alkyl, unsubstituted C₃-C₁₀ cycloalkyl, and substituted C₃-C₁₀ cycloalkyl;
R₈ and R₉ are independently selected from H, unsubstituted C₁-C₈ alkyl, substituted C₁-C₈ alkyl, unsubstituted C₃-C₁₀ cycloalkyl, O-substituted C₁-C₈ alkyl, O-unsubstituted C₁-C₈ alkyl, CH₃O(CH₂CH₂)ₙCH₂CH₂-, and HO(CH₂CH₂)ₙCH₂CH₂-;
R₁₀ is 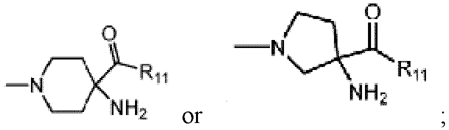
R₁₁ is OR₁₂ or NR₁₃R₁₄;
R₁₂ is selected from H, unsubstituted C₁-C₂₄ alkyl, substituted C₁-C₂₄ alkyl, O-substituted C₁-C₂₄ alkyl, O-unsubstituted C₁-C₂₄ alkyl, CH₃O(CH₂CH₂)ₙCH₂CH₂-, and HO(CH₂CH₂)ₙCH₂CH₂-;
R₁₃ and R₁₄ are independently selected from H, unsubstituted C₁-C₂₄ alkyl, substituted C₁-C₂₄ alkyl, O-substituted C₁-C₂₄ alkyl, O-unsubstituted C₁-C₂₄ alkyl, CH₃O(CH₂CH₂)ₙCH₂CH₂-, and HO(CH₂CH₂)ₙCH₂CH₂-; and
n is an integer from 0 to 100;
the pH adjusting agent is hydrochloric acid; and pH of the pharmaceutical composition is 3.0-5.0.

2. The pharmaceutical composition of claim 1, wherein in the compound of formula (I):
R₁, R₂, and R₃ are independently selected from the group consisting of H, CN, Cl, F, unsubstituted C₁-C₈ alkyl, substituted C₁-C₈ alkyl, unsubstituted C₃-C₁₀ cycloalkyl, and substituted C₃-C₁₀ cycloalkyl;
R₄ is selected from H, unsubstituted C₁-C₈ alkyl, substituted C₁-C₈ alkyl, unsubstituted C₃-C₁₀ cycloalkyl, and substituted C₃-C₁₀ cycloalkyl;
R₅ and R₆ are independently selected from H, unsubstituted C₁-C₈ alkyl, substituted C₁-C₈ alkyl, unsubstituted C₃-C₁₀ cycloalkyl, substituted C₃-C₁₀ cycloalkyl, unsubstituted aryl, substituted aryl, unsubstituted heterocyclyl, and substituted heterocyclyl;
R₇ is selected from H, unsubstituted C₁-C₈ alkyl, substituted C₁-C₈ alkyl, unsubstituted C₃-C₁₀ cycloalkyl, and substituted C₃-C₁₀ cycloalkyl;
R₈ and R₉ are independently selected from H, unsubstituted C₁-C₈ alkyl, substituted C₁-C₈ alkyl, unsubstituted C₃-C₁₀ cycloalkyl, O-substituted C₁-C₈ alkyl, and O-unsubstituted C₁-C₈ alkyl;
R₁₀ is 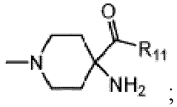
R₁₁ is OR₁₂ or NR₁₃R₁₄;
R₁₂ is selected from H, unsubstituted C₁-C₂₄ alkyl, substituted C₁-C₂₄ alkyl, O-substituted C₁-C₂₄ alkyl, O-unsubstituted C₁-C₂₄ alkyl, CH₃O(CH₂CH₂)ₙCH₂CH₂-, and HO(CH₂CH₂)ₙCH₂CH₂-;
R₁₃ and R₁₄ are independently selected from H, unsubstituted C₁-C₂₄ alkyl, substituted C₁-C₂₄ alkyl, O-substituted C₁-C₂₄ alkyl, O-unsubstituted C₁-C₂₄ alkyl, CH₃O(CH₂CH₂)ₙCH₂CH₂-, and HO(CH₂CH₂)ₙCH₂CH₂-; and
n is an integer from 0 to 100.

3. The pharmaceutical composition of claim 2, wherein in the compound of formula (I):
R₁, R₂ and R₃ are independently selected from H, CN, Cl, F, unsubstituted C₁-C₄ alkyl, substituted C₁-C₄ alkyl, unsubstituted C₃-C₈ cycloalkyl, and substituted C₃-C₈ cycloalkyl;
R₄ is selected from unsubstituted C₁-C₄ alkyl, substituted C₁-C₄ alkyl, unsubstituted C₃-C₈ cycloalkyl, and substituted C₃-C₈ cycloalkyl;
R₅ and R₆ are independently selected from H, unsubstituted C₁-C₄ alkyl, substituted C₁-C₄ alkyl, unsubstituted C₃-C₈ cycloalkyl, substituted C₃-C₈ cycloalkyl, unsubstituted aryl, substituted aryl, unsubstituted heterocyclyl, and substituted heterocyclyl;
R₇ is selected from unsubstituted C₁-C₄ alkyl, substituted C₁-C₄ alkyl, unsubstituted C₃-C₈ cycloalkyl, and substituted C₃-C₈ cycloalkyl;
R₈ and R₉ are independently selected from H, unsubstituted C₁-C₄ alkyl, substituted C₁-C₄ alkyl, O-substituted C₁-C₄ alkyl, and O-unsubstituted C₁-C₄ alkyl;
R₁₀ is 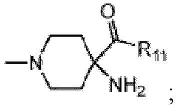
R₁₁ is selected from OR₁₂ and NR₁₃R₁₄;
R₁₂ is selected from H, unsubstituted C₁-C₁₂ alkyl, substituted C₁-C₁₂ alkyl, O-substituted C₁-C₁₂ alkyl, O-unsubstituted C₁-C₁₂ alkyl, CH₃O(CH₂CH₂)ₙCH₂CH₂-, and HO(CH₂CH₂)ₙCH₂CH₂-;
R₁₃ and R₁₄ are independently selected from H, unsubstituted C₁-C₁₂ alkyl, substituted C₁-C₁₂ alkyl, O-substituted C₁-C₁₂ alkyl, O-unsubstituted C₁-C₁₂ alkyl, CH₃O(CH₂CH₂)ₙCH₂CH₂-, and HO(CH₂CH₂)ₙCH₂CH₂-; and
n is an integer from 0 to 50.

4. The pharmaceutical composition of claim 2, wherein in the compound of formula (I):
R₁, R₂, and R₃ are independently selected from H, Cl, F, methyl, ethyl, propyl, and isopropyl;
R₄ is selected from methyl, ethyl, propyl, isopropyl, cyclopropyl, cyclobutyl, cyclopentyl, and cyclohexyl;
R₅ and R₆ are independently selected from H, methyl, ethyl, n-propyl, isopropyl, cyclopropyl, cyclobutyl, cyclopentyl, cyclohexyl, and phenyl;
R₇ is selected from methyl, ethyl, propyl, isopropyl, cyclopropyl, cyclobutyl, cyclopentyl, and cyclohexyl;
R₈ and R₉ are independently selected from H, methyl, ethyl, propyl, and isopropyl;
R₁₀ is 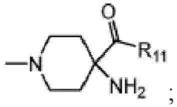
R₁₁ is selected from OR₁₂ and NR₁₃R₁₄;
R₁₂ is selected from H, methyl, ethyl, n-propyl, and isopropyl; and
R₁₃ and R₁₄ are independently selected from H, methyl, ethyl, n-propyl, isopropyl, cyclopropyl, cyclobutyl, cyclopentyl, and cyclohexyl.

5. The pharmaceutical composition of claim 2, wherein in the compound of formula (I):
R₁, R₂, R₃, R₅, R₈, and R₉ are H; R₄ is methyl; R₆ is phenyl; R₇ is isopropyl;
R₁₀ is 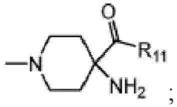
R₁₁ is OR₁₂; R₁₂ is H; and R₁₃, R₁₄, and n are absent, and the compound of formula (I) is a compound as represented by formula (IV):

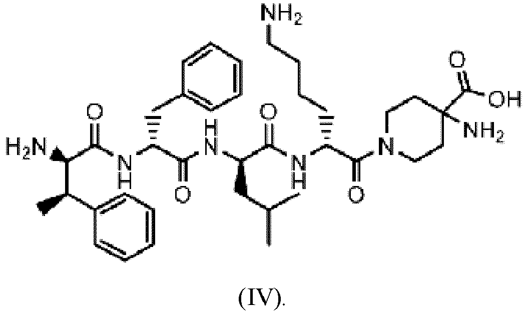

6. The pharmaceutical composition of claim 2, wherein in the compound of formula (I):
R₁, R₂, R₃, R₅, R₈, and R₉ are H; R₄ is methyl; R₆ is phenyl; R₇ is isopropyl;
R₁₀ is 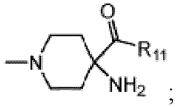
R₁₁ is OR₁₂; R₁₂ is Me; and R₁₃, R₁₄, and n are absent, and the compound of formula (I) is a compound as represented by formula (V):

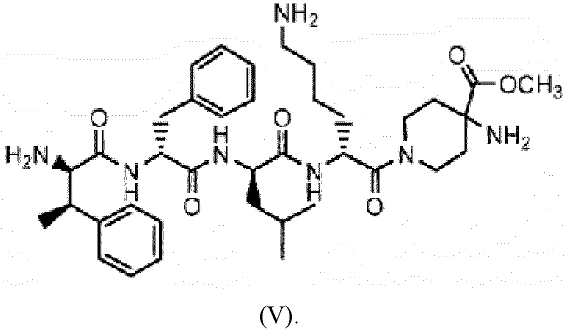

7. The pharmaceutical composition of claim 1, wherein in the compound of formula (I):
R₁, R₂, and R₃ are independently selected from the group consisting of H, CN, Cl, F, unsubstituted C₁-C₈ alkyl, substituted C₁-C₈ alkyl, unsubstituted C₃-C₁₀ cycloalkyl, and substituted C₃-C₁₀ cycloalkyl;
R₄ is selected from H, unsubstituted C₁-C₈ alkyl, substituted C₁-C₈ alkyl, unsubstituted C₃-C₁₀ cycloalkyl, and substituted C₃-C₁₀ cycloalkyl;
R₅ and R₆ are independently selected from H, unsubstituted C₁-C₈ alkyl, substituted C₁-C₈ alkyl, unsubstituted C₃-C₁₀ cycloalkyl, substituted C₃-C₁₀ cycloalkyl, unsubstituted aryl, substituted aryl, unsubstituted heterocyclyl, and substituted heterocyclyl;
R₇ is selected from H, unsubstituted C₁-C₈ alkyl, substituted C₁-C₈ alkyl, unsubstituted C₃-C₁₀ cycloalkyl, and substituted C₃-C₁₀ cycloalkyl;
R₈ and R₉ are independently selected from H, unsubstituted C₁-C₈ alkyl, substituted C₁-C₈ alkyl, unsubstituted C₃-C₁₀ cycloalkyl, O-substituted C₁-C₈ alkyl, O-unsubstituted C₁-C₈ alkyl, CH₃O(CH₂CH₂)ₙCH₂CH₂-, and HO(CH₂CH₂)ₙCH₂CH₂-;
R₁₀ is 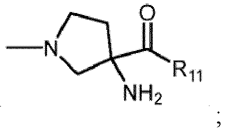
R₁₁ is OR₁₂ or NR₁₃R₁₄;
R₁₂ is selected from H, unsubstituted C₁-C₂₄ alkyl, substituted C₁-C₂₄ alkyl, O-substituted C₁-C₂₄ alkyl, O-unsubstituted C₁-C₂₄ alkyl, CH₃O(CH₂CH₂)ₙCH₂CH₂-, and HO(CH₂CH₂)ₙCH₂CH₂-;
R₁₃ and R₁₄ are independently selected from H, unsubstituted C₁-C₂₄ alkyl, substituted C₁-C₂₄ alkyl, O-substituted C₁-C₂₄ alkyl, O-unsubstituted C₁-C₂₄ alkyl, CH₃O(CH₂CH₂)ₙCH₂CH₂-, and HO(CH₂CH₂)ₙCH₂CH₂-; and
n is an integer from 0 to 100.

8. The pharmaceutical composition of claim 7, wherein in the compound of formula (I):
R₁, R₂ and R₃ are independently selected from H, CN, Cl, F, unsubstituted C₁-C₄ alkyl, substituted C₁-C₄ alkyl, unsubstituted C₃-C₈ cycloalkyl, and substituted C₃-C₈ cycloalkyl;
R₄ is selected from unsubstituted C₁-C₄ alkyl, substituted C₁-C₄ alkyl, unsubstituted C₃-C₈ cycloalkyl, and substituted C₃-C₈ cycloalkyl;
R₅ and R₆ are independently selected from H, unsubstituted C₁-C₄ alkyl, substituted C₁-C₄ alkyl, unsubstituted C₃-C₈ cycloalkyl, substituted C₃-C₈ cycloalkyl, unsubstituted aryl, substituted aryl, unsubstituted heterocyclyl, and substituted heterocyclyl;
R₇ is selected from unsubstituted C₁-C₄ alkyl, substituted C₁-C₄ alkyl, unsubstituted C₃-C₈ cycloalkyl, and substituted C₃-C₈ cycloalkyl;
R₈ and R₉ are independently selected from H, unsubstituted C₁-C₄ alkyl, substituted C₁-C₄ alkyl, O-substituted C₁-C₄ alkyl, O-unsubstituted C₁-C₄ alkyl, CH₃O(CH₂CH₂)ₙCH₂CH₂-, and HO(CH₂CH₂)ₙCH₂CH₂-;
R₁₀ is 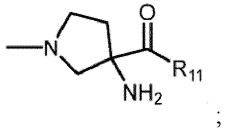
R₁₁ is selected from OR₁₂ and NR₁₃R₁₄;
R₁₂ is selected from H, unsubstituted C₁-C₁₂ alkyl, substituted C₁-C₁₂ alkyl, O-substituted C₁-C₁₂ alkyl, O-unsubstituted C₁-C₁₂ alkyl, CH₃O(CH₂CH₂)ₙCH₂CH₂-, and HO(CH₂CH₂)ₙCH₂CH₂-;
R₁₃ and R₁₄ are independently selected from H, unsubstituted C₁-C₁₂ alkyl, substituted C₁-C₁₂ alkyl, O-substituted C₁-C₁₂ alkyl, O-unsubstituted C₁-C₁₂ alkyl, CH₃O(CH₂CH₂)ₙCH₂CH₂-, and HO(CH₂CH₂)ₙCH₂CH₂-; and
n is an integer from 0 to 50.

9. The pharmaceutical composition of claim 7, wherein in the compound of formula (I):
R₁, R₂, and R₃ are independently selected from H, Cl, F, methyl, ethyl, propyl, and isopropyl;
R₄ and R₇ are independently selected from methyl, ethyl, propyl, isopropyl, cyclopropyl, cyclobutyl, cyclopentyl, and cyclohexyl;
R₅ and R₆ are independently selected from H, methyl, ethyl, n-propyl, isopropyl, cyclopropyl, cyclobutyl, cyclopentyl, cyclohexyl, and phenyl;
R₈ and R₉ are independently selected from H, methyl, ethyl, propyl, and isopropyl;
R₁₀ is 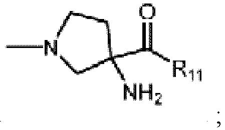
R₁₁ is selected from OR₁₂ and NR₁₃R₁₄;
R₁₂ is selected from H, methyl, ethyl, n-propyl, and isopropyl; and
R₁₃ and R₁₄ are independently selected from H, methyl, ethyl, n-propyl, isopropyl, cyclopropyl, cyclobutyl, cyclopentyl, and cyclohexyl.

10. The pharmaceutical composition of claim 7, wherein in the compound of formula (I):
R₁, R₂, R₃, R₅, R₈, and R₉ are H; R₄ is methyl; R₆ is phenyl; R₇ is isopropyl;
R₁₀ is 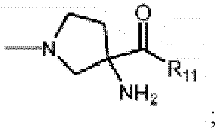
R₁₁ is OR₁₂; R₁₂ is H; and R₁₃, R₁₄, and n are absent, and the compound of formula (I) is a compound as represented by formula (II):

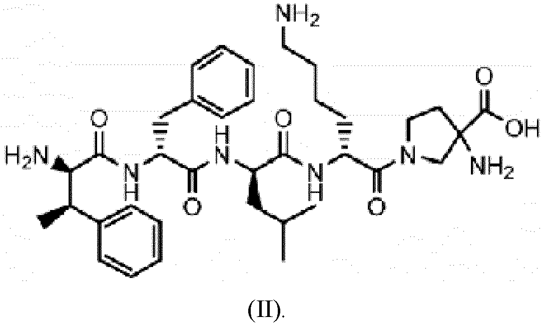

11. The pharmaceutical composition of claim 7, wherein in the compound of formula (I):
R₁, R₂, R₃, R₅, R₈, and R₉ are H; R₄ is methyl; R₆ is phenyl; R₇ is isopropyl;
R₁₀ is 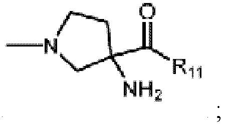
R₁₁ is OR₁₂; R₁₂ is Me; and R₁₃, R₁₄, and n are absent, the compound of formula (I) is a compound as represented by formula (III):

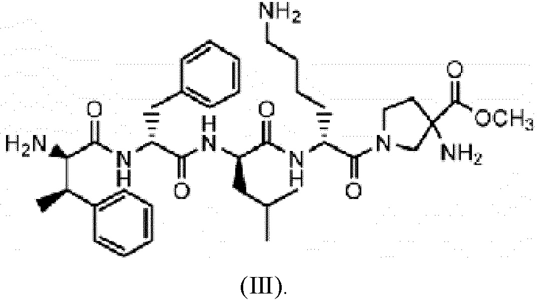

12. The pharmaceutical composition of claim 1, wherein the pharmaceutically acceptable salt is an acid addition salt; alternatively, the pharmaceutically acceptable salt is selected from one of acetate, hydrobromide, phosphate, sulfate, formate, citrate, mesylate, maleate, malate, succinate, and fumarate.

13. A pharmaceutical composition of a kappa opioid receptor agonist, the pharmaceutical composition comprising an acetate salt of a compound represented by formula (II), optionally an osmotic pressure regulator, and a pH adjusting agent and water for injection;
wherein the compound represented by formula (II) is: 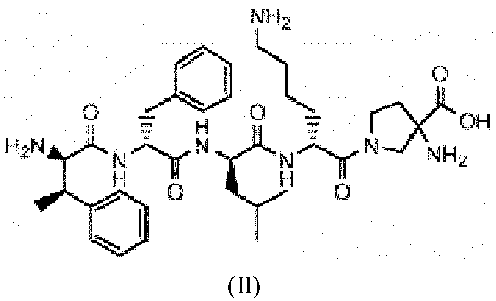 the pH adjusting agent is hydrochloric acid; and pH of the pharmaceutical composition is 3.0-5.0.

14. The pharmaceutical composition of any one of claims 1 to 13, wherein content of the pH adjusting agent is such that the pH of the pharmaceutical composition is 3.0-4.5, 3.5-5.0, or 3.5-4.5, alternatively, 4.0.

15. The pharmaceutical composition of any one of claims 1 to 14, wherein content of the compound represented by formula (I) or the compound represented by formula (II) is 0.01 mg/mL-50 mg/mL; or 0.01 mg/mL-0.1 mg/mL, 0.1 mg/mL-0.2 mg/mL, or 0.2 mg/mL-50 mg/mL; or 0.01 mg/mL, 0.1 mg/mL, 0.2 mg/mL, or 50 mg/mL.

16. The pharmaceutical composition of any one of claims 1 to 15, wherein the osmotic pressure regulator is selected from one or more of sodium chloride, mannitol, sorbitol, and glucose.

17. The pharmaceutical composition of any one of claims 1 to 16, wherein the content of the osmotic pressure regulator is 0.25 mg/mL-9 mg/mL.

18. The pharmaceutical composition of any one of claims 1 to 17, wherein the pharmaceutical composition is an injection solution or a freeze-dried injection.

19. A method for preparing the pharmaceutical composition of any one of claims 1 to 18, comprising:
dissolving an optional osmotic pressure regulator in water for injection to obtain Solution 1;
mixing the Solution 1 with an active ingredient to obtain Solution 2;
adjusting pH of the Solution 2 to 3.0-5.0 with hydrochloric acid solution to obtain Solution 3; and
making the Solution 3 up to volume, filtering, filling and sealing, and sterilizing, to obtain the pharmaceutical composition.

20. A kappa opioid receptor agonist, which is an acetate salt of a compound as represented by formula (II):

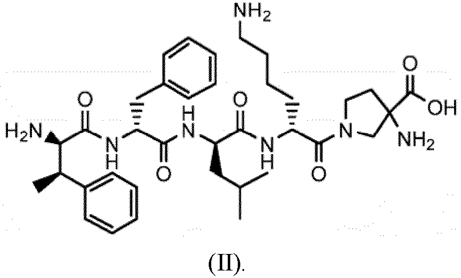

21. The pharmaceutical composition of any one of claims 1 to 18, or the kappa opioid receptor agonist of claim 20, for use in preventing, treating, or treating and preventing a disease associated with a kappa opioid-like substance receptor.

22. Use of the pharmaceutical composition of any one of claims 1 to 18 or the kappa opioid receptor agonist of claim 20 in the preparation of a medicament for preventing, treating, or treating and preventing a disease associated with a kappa opioid-like substance receptor.

23. A method for preventing, treating, or treating and preventing a disease associated with a kappa opioid-like substance receptor, comprising administering to an individual in need thereof a therapeutically effective amount of the pharmaceutical composition of any one of claims 1 to 18 or the kappa opioid receptor agonist of claim 20.

24. The pharmaceutical composition or the kappa opioid receptor agonist for use of claim 21, the use of claim 22, or the method of claim 23, wherein the disease associated with a kappa opioid-like substance receptor is selected from one or more of pain, cardiovascular disease, pruritus, nausea, inflammation, spinal anesthesia, cough, stroke, hypoxic pulmonary hypertension, multiple sclerosis, addiction, and post-traumatic cartilage degeneration.
